# EUROPEAN PATENT APPLICATION

(11) **EP 4 640 054 A1**
(43) Date of publication of application: **29.10.2025**
(21) Application number: 23904816.8
(22) Date of filing: 22.12.2023
(51) Int. Cl.: A01N 63/32, A01N 43/16, C12N 1/16, C12P 1/02, A01N 25/02, A01N 25/12

(54) **COMPOSITION TO PROMOTE THE MULTIPLICATION AND SURVIVAL OF MICROORGANISMS, METHOD OF TREATING PLANTS AND USE OF THE COMPOSITION IN AGRICULTURAL BIOPESTICIDES**

(30) Priority: 23.12.2022 BR 102022026525; 21.12.2023 BR 102023027177
(71) Applicant: Yessinergy Holding S.A., 13049-254 Campinas (BR)
(72) Inventor: ABOU NEHMI FILHO, Victor, São Paulo - São Paulo CEP: 04644-000 (BR); LOPES FERREIRA, Leonnardo, Osvaldo Cruz São Paulo CEP: 17700-000 (BR)
(74) Representative: Studio Torta S.p.A.
(86) International application number: PCT/BR2023/050494
(87) International publication number: WO 2024/130376

(57) **Abstract**

The present invention relates to the use of the cell extract of *Saccharomyces cerevisiae,* a by-product of alcohol production, in a composition to sustainably promote the multiplication and survival of bacteria and fungi for use in agriculture or agro-industry. Among the microorganisms enhanced by this invention, bacteria that include the genera *Bacillus spp.* can be cited., Rhizobium spp, Azospirillum spp, Azotobacter, Pseudomonas spp, Allorhizobium spp, Azorhizobium, Bradyrhizobium and Streptomyces spp, the fungi Metarhizium spp and Beauveria spp and Trichoderma spp, in addition to the mycorrhizal fungi Glomus spp, Rhizophagus spp, Funneliformis spp, Amanita spp, Boletus spp and Laccaria spp.

Said composition promote the multiplication and survival of target microorganisms comprising fructo-oligosaccharides (FOS) and cell extract of *Saccharomyces cerevisae,* and also comprises a mass concentration of 1.3 and 1.6 Betaglucans ranging from 1% to 15%, said betaglucans coming from the cell extract or already added in the form of cell wall.

## Description

### FIELD OF APPLICATION

The present invention refers to the use of cell extract of *Saccharomyces cerevisiae,* a co-product from alcohol production, in a composition to sustainably promote the multiplication and survival of bacteria and fungi for use in agriculture or for the agro-industry. Among the target microorganisms, enhanced by this invention, it can be cited fungi or bacteria that involve the genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp.* and *Trichoderma spp.,* in addition to the mycorrhizal fungi *Glomus spp., Rhizophagus spp., Funneliformis spp., Amanita spp., Boletus spp.* and *Laccaria spp..*

### PRIOR ART

For some years now, agriculture has been witnessing a strong trend towards replacing chemical pesticides, used on plantations to combat pests and diseases, with biological pesticides, also known as biodefensives.

These biological defensives are made up of dozens of species of microorganisms and insects, which have been used to combat pests and pathogens that attack agricultural crops, such as flowers, fruits, vegetables, legumes, grains, etc..

There are two basic categories of biodefensives. One of them is foliar, that is, products that are sprayed on leaves and fruits, acting on the aerial microbiota of plants. The other category is subterranean, that is, products that are applied to the soil and act on the microbiota that lives in the rhizosphere of plants.

In both categories, the bacteria genus *Bacillus spp., Trichoderma spp.* and *Beauveria spp.* have been widely used to combat undesirable or pathogenic bacteria and fungi.

The basic process for its use is the multiplication of desired species in biofactories having a controlled environment, for supply to agricultural producers in packages containing a standardized concentration, generally around 10⁹ CFU/ml.

The biodefensive, at a concentration of approximately 10⁹ CFU/ml, is then diluted and sprayed on the plantations.

The plants produce sugary sweats capable of keeping a symbiotic microbiota alive, which helps protect their leaves and fruits against the attack of pathogens. The enhanced bacteria and fungi stand out in this symbiotic microbiota, due to their ability to combat pathogenic fungi and bacteria.

Bacteria of *Bacillus* genus produce bacteriocins, which are effective against all species of pathogenic bacteria, in addition to producing other bioactive substances that delay the hatching of spores of pathogenic fungi. In other words, bacteria of *Bacillus* genus prevent the growth of populations of pathogenic fungi and bacteria.

However, their efficiency depends on the size of the population of pathogens to be controlled. In nature, the autochthonous populations of *Bacillus* bacteria, existing on the surface of plants, are sufficient to control the incidence of pathogens, whose populations are usually small, due to the diversity of plants existing in the location. However, against massive attacks of pathogens, such as those that occur in agricultural monocultures, large populations of *Bacillus* bacteria are needed, much larger than the autochthonous populations.

With the aim of significantly increasing the populations of bacteria and fungi on the surface of plants, agricultural producers are spraying biodefensives containing high concentrations of these microorganisms produced in biofactories or "on farm".

In biofactories, technicians usually multiply microorganisms in a culture medium composed of sugar, a source of protein (usually cell extract) and minerals. This culture medium is used to feed any microorganism, whether beneficial or pathogenic. This is why biofactories maintain strict sterilization standards for their equipment. However, this culture medium cannot be used to spray plants, as it would serve as food for the propagation of all types of existing microorganisms, including pathogenic ones.

The solution adopted is to spray high concentrations of the desired microorganisms, diluted only in water, without the addition of nutrients.

However, the amount of sweat produced by plants is insufficient to sustain populations much larger than the autochthonous ones, which is why the increased populations of bacteria or fungi have a short lifespan and die from starvation.

This is the same problem faced by probiotics ingested by humans. Daily doses must be consumed if the goal is to maintain increased populations of certain species.

Those skilled in the art already use some types of selective oligosaccharides as nutrients for bacteria and fungi. The document WO 2014152174 uses glucans as nutrients for *Bacillus* bacteria in plants. Documents CN 112939673, CN 113367138 and CN 114380629, on the other hand, use fructo-oligosaccharides (FOS) in compositions for *Bacillus* in plants.

Green biotechnology is a branch of biotechnology with applications in agricultural processes mainly to reduce dependence on fertilizers, pesticides and other agrochemicals. An example is the use of plant growth promoting bacteria (PGPB) that has gained importance in the area of sustainable agriculture. PGPB are a group of microorganisms capable of conferring beneficial effects on plant growth and development, without causing harm to the host. PGPB bacteria belong to a broader group, PGPM (plant growth promoting microbes), which includes other microbes besides bacteria, such as plant growth promoting fungi (PGPF). PGPB that colonize the rhizosphere are sometimes called plant growth promoting rhizobacteria (PGPR).

These microorganisms can stimulate plant growth as a consequence of nitrogen fixation, phytohormone production, increased mineral availability and also phytoremediation.

The importance of PGPB is its contribution to crop improvement.

The present invention relates to a composition capable of promoting the multiplication and survival of these plant growth promoting microorganisms (PGPB).

### OBJECTIVE OF THE INVENTION

The objective of the invention is to provide a product capable of enhancing the effect of biodefensives based on bacteria that include the genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp.* and *Trichoderma spp.,* in addition to the mycorrhizal fungi *Glomus spp., Rhizophagus spp., Funneliformis spp., Amanita spp., Boletus spp.* and *Laccaria spp.,* providing a sustainable solution.

### SOLUTION TO SOLVE THE PROBLEM

The present invention aims to solve this problem. It aims to increase the populations of target microorganisms and prolong their survival, reducing the number of sprayings required during the crop cycle. This saves costs and provides greater protection for plants, due to the larger population of target microorganisms maintained on their surface, whether aerial or underground.

The solution is to use FOS as a food energy source, since only the target microorganisms can digest it. FOS must also be combined with a protein source that does not contain sugars, which can be used by other bacteria and fungi, competing with the target microorganisms. This is the case of the cell extract of *Saccharomyces cerevisae,* which contains only traces of trehalose and glycogen.

Most agricultural pesticides, whether chemical or biological, are used preventively. The molecules of chemical pesticides are stable and have a much longer half-life compared to the bacteria in biopesticides. The latter, due to the starvation of the sprayed bacteria, usually have a survival rate of only a few days. Therefore, it is necessary to repeat the spraying weekly.

A second problem faced by biodefensives is that the recommended dosage is not always technically ideal. In other words, since these are expensive products, the dosage recommended by technicians and manufacturers is the one that offers the best benefit/cost ratio. However, if there were no cost limitations, it is likely that most biodefensives would have better performance if they were used in higher dosages.

A third and final problem faced by biodefensives is that, due to storage problems or manufacturing errors, these products may contain a concentration lower than the standard 10⁹ CFU/ml. For example, they may contain a concentration of 10⁸, which is 10 times lower. Therefore, to obtain the same effect, using a lower quality product, such as the one in the example, it would be necessary to apply a dose ten times higher.

The solution to these three problems, according to the present invention, was to develop a nutritional composition that could only be consumed by the target microorganisms. This composition would need to contain energy, proteins, vitamins and mineral salts to sustain and/or significantly increase the population of these good bacteria on the surface of the plants. To be technically and economically viable, this solution should solve the three problems reported above, that is, increase the survival of the increased population of target microorganisms, allow for technically better effects of the product to be achieved, without compromising economic performance, and compensate for any low quality of products with a lower concentration of CFUs (Colony Forming Units), which is a unit of measurement used to estimate the number of viable bacteria or fungi.

If the food used is rapidly metabolized by bacteria, its effect will be a rapid explosion of bacteria, but of short duration. On the other hand, if the food has slow release, it will not cause an exaggerated growth of the bacterial population, but will provide greater longevity. In most cases, this second option is the most desirable, because it allows for an increase in the interval between sprayings, which provides longer-lasting protection for the cultured crops.

In order to multiply and stay alive, microorganisms need sugars, especially glucose, proteins (amino acids), minerals and vitamins. All of these must be consumed in constant proportions. According to the Law of the Minimum, or Law of Mitsherlich, the food whose supply is most limited will determine the performance of the bacterial population, in other words, its growth or mortality rate.

FOS (fructo-oligosaccharides) is a source of glucose that can only be fermented, or metabolized, by microorganisms that produce the invertase enzyme. Thus, FOS is the ideal energy source to be consumed exclusively by the target microorganisms, as they produce this enzyme. By using FOS, it is possible to calculate exactly the desired growth rate of the bacterial population, as all the glucose will be used only by them, without competition from other microorganisms. Likewise, depending on the average size of the FOS oligomers used, it is possible to calculate the longevity of the bacterial population. Because the larger the average size of the molecular chains of the oligomers used, the longer it will take to ferment them, thus increasing the survival of the population, due to the slower release of the energy source.

The solution of the present invention is to develop a composition containing cell extract (cytoplasm and nucleotides) of *Saccharomyces cerevisae,* to serve as a source of proteins, vitamins and minerals, and containing FOS, to serve as a source of glucose, having slow release and being exclusive for fermentation of target microorganisms.

### UNUSUAL EFFECT

The present composition managed to increase the population of microorganisms and their longevity, reducing at the same time the costs of inputs for agriculture that uses biodefensives, for two reasons: first, because its effectiveness reduced the number of necessary sprayings of biodefensives during the crop cycle, and second, because the cost of the composition of the present invention is lower than that of biodefensives.

### SUMMARY OF THE INVENTION

The present invention describes a composition and a treatment method to promote the multiplication and survival of microorganisms, said composition comprising fructo-oligosaccharides (FOS) and cell extract of *Saccharomyces cerevisae,* also comprising a mass concentration of 1.3 and 1.6 Betaglucans ranging from 1% to 15%, wherein said betaglucans come from the cell extract, or have already been added in the form of cell wall.

### DETAILED DESCRIPTION

The present invention relates to a composition of fructo-oligosaccharides (FOS) and cell extract of *Saccharomyces cerevisae,* with the objective of promoting the multiplication and survival of microorganisms, such as fungi and bacteria that involve the genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp.* and *Trichoderma spp.,* in addition to the mycorrhizal fungi *Glomus spp., Rhizophagus spp., Funneliformis spp., Amanita spp., Boletus spp.* and *Laccaria spp..*

These target microorganisms may be autochthonous or may have been sprayed onto the plant.

The present invention aims to reduce the dosage and number of sprayings with target microorganisms during the crop cycle. This results in cost savings and greater protection for the plants, due to the larger population of target microorganisms maintained on their surface, whether aerial or underground.

The composition of the present invention may be presented in powder or liquid form. In powder form, it will have a moisture content of less than 5% and may be diluted in water for spraying onto the plants. In liquid form, the product must have 20% and 50% dry matter.

According to Table 1 below, the basic composition of the present invention, based on dry matter mass, is 10% to 90% fructo-oligosaccharides (FOS) and 10% to 90% cell extract of *Saccharomyces cerevisae.* In addition, the concentration of 1,3 and 1,6 Betaglucans can vary between 1% to 15%, wherein said betaglucans come from the cell extract, or have already been added in the form of cell wall.

**TABLE 1**

| **Composition components** | **Minimum by mass** | **Maximum by mass** |
|---|---|---|
| FOS | 10% | 90% |
| Cell extract of *Saccharomyces cerevisiae* | 10% | 90% |
| 1,3 and 1,6 Betaglucans | 1% | 15% |

According to Table 2 below, a preferred form of the composition according to the invention, based on dry matter, comprises from 15 to 90% FOS and from 15% to 90% cell extract of *Saccharomyces cerevisae.* In addition, the concentration of 1,3 and 1,6 Betaglucans may vary between 2% and 15%, wherein such betaglucans come from the cell extract, or have already been added in the form of cell wall.

**TABLE 2**

| **Composition components** | **Minimum by mass** | **Maximum by mass** |
|---|---|---|
| FOS | 15% | 90% |
| Cell extract of *Saccharomyces cerevisiae* | 15% | 90% |
| 1,3 and 1,6 Betaglucans | 2% | 15% |

According to Table 3 below, an even more preferred form of the composition of the product according to the invention, based on dry matter, is 20 to 90% FOS and 20% to 90% cell extract of *Saccharomyces cerevisae.* In addition, the concentration of 1,3 and 1,6 Betaglucans must vary between 2% and 15%, wherein said betaglucans come from the cell extract, or have already been added in the form of cell wall.

**TABLE 3**

| **Composition components** | **Minimum by mass** | **Maximum by mass** |
|---|---|---|
| FOS | 20% | 90% |
| Cell extract of *Saccharomyces cerevisiae* | 20% | 90% |
| 1,3 and 1,6 Betaglucans | 2% | 15% |

In an alternative form, the basic composition of this invention may be enriched with betaglucans and minerals.

The FOS of the present invention is the one that contains the oligomers GF2 (one glucose G linked to two fructoses F), GF3, GF4, GF5 and GF6. Preferably, for the present invention, the FOS to be used should be the one that contains the highest possible proportion of oligomers GF4, GF5 and GF6, which are more slowly metabolized.

The cell extract of *Saccharomyces cerevisae* chosen was an industrial co-product from ethanol production, after undergoing the cell wall production process using protease hydrolysis. During the cell wall production process, yeast undergoes food stress to eliminate trehalose and glycogen in the cytoplasm by converting them into ethanol. After this process, the cytoplasm is practically free of energy sources that can be metabolized, which prevents the growth of undesirable microorganisms that could compete with the target microorganisms. This extract is also richer in betaglucans than the cell extract from primary fermentation, such as that from breweries and yeast factories for bread making. Betaglucans play an important role in the immunostimulation of plants, which leads them to produce and release more vesicles of repellent substances, which help protect crops by combining their effects with those of the action of target microorganisms.

The cell extract of *Saccharomyces cerevisae* of the present invention is from alcoholic fermentation. According to the invention, the cell extract must come from the production of cell walls, by enzymatic hydrolysis with protease, from yeasts that are surplus from production of fuel ethanol, i.e., the residue from the manufacture of ethanol. Preferably, this must have been extracted after the application of nutritional stress to the yeasts, for the transformation of trehalose and glycogen contained in the cytoplasm into ethanol. The extract thus produced will be rich in micro-particulate betaglucans, which escape from centrifugation, and poor in sugar sources, which can be used by microorganisms that can compete with the target microorganisms. The 1,3 and 1,6 betaglucans are important plant immunostimulants and their effect adds to that of the target microorganisms, in protecting plants against pathogens. The cell extract of *Saccharomyces cerevisae* of the composition must not contain trehalose or glycogen. However, it is desirable that it contains 1,3 and 1,6 Betaglucans.

According to a method of application of the product comprising the composition of the present invention, it must be mixed with the biodefensive containing the target microorganisms, so that each spray droplet is made up of the aqueous solution of the composition of this invention, inoculated with the target microorganisms of the biodefensive.

According to another method of application of the present invention, the product of this invention can also be sprayed alone on plants, without being mixed with biodefensives containing target microorganisms, since most plants have autochthonous populations of these good bacteria, which will benefit from the composition of this invention, through the increase in their population.

The recommended dosage for application of the composition according to the invention on plants or in the soil is variable, since it depends on the dry matter content of the composition and the desired effects. For the purposes of this invention, the dosage of the composition in dry matter mass, recommended for its application by spraying on plants or soil, can vary between 0.20 kg/ha and 1.00 kg/ha.

The method of treating plants with the composition employs the invention mixed with the biodefensive containing microorganisms, fungi, or bacteria chosen from the group comprising genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp.* and *Trichoderma spp.,* in addition to the mycorrhizal fungi *Glomus spp., Rhizophagus spp., Funneliformis spp., Amanita spp., Boletus spp.* and *Laccaria spp.*

The composition can also be sprayed alone on plants, without being mixed with biodefensives containing said microorganisms, fungi, or bacteria (chosen from the group comprising genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp.* and *Trichoderma spp.,* in addition to the mycorrhizal fungi *Glomus spp., Rhizophagus spp., Funneliformis spp., Amanita spp., Boletus spp.* and *Laccaria spp.*)*.*

The present invention can be employed or used in agriculture or agro-industry, to stimulate the growth and viability of microbial strains of bacteria that involve the genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp.* and *Trichoderma spp.,* in addition to the mycorrhizal fungi *Glomus spp., Rhizophagus spp., Funneliformis spp., Amanita spp., Boletus spp.* and *Laccaria spp.*

The following examples are intended to better elucidate the scope of the invention, as well as to make it clearer, more precise and reproducible, and should not be used to limit the invention.

### EXAMPLES OF USE

### EXAMPLE 1

Example 1 was performed to demonstrate the different effects of several possible compositions of this invention on the population of bacteria of genus *Bacillus,* from a commercial formulation containing *Bacillus subtilis,* as the proportions of FOS and cell extract of *Saccharomyces cerevisae* vary.

The commercial formulation used was Bio Imune, manufactured by the company Vittia, composed of bacteria *Bacillus subtilis* BV02. The product is recommended for combating the fungus *Phakospsora pachrhizi,* which causes Asian rust disease in soybean crops. The recommended use is 1.0 liter of product per ha/application, for a total of 4 applications per crop cycle. The dilution of the solution is 1:200, in order to make applications of 200 I/ha. The concentration of the product announced by the manufacturer was 3 × 10⁹.

Since it is practically impossible to accurately measure the population of *Bacillus* bacteria on plant leaves, it was decided to dilute the Bio Imune product in the laboratory and culture the bacteria *in vitro,* in a culture media containing six different compositions of FOS and cell extract of *Saccharomyces cerevisae,* within the scope of the present invention. A seventh treatment was the control, which did not receive any composition of this invention. All treatments contained 400 ml of liquid volume.

The FOS used to make the treatment compositions was YES FOS syrup, manufactured by the company Yessinergy, having a concentration of 88% FOS and dry matter of 32%. The cell extract of *Saccharomyces cerevisae* was also produced commercially by the company Yessinergy and contained 41% crude protein, 10% 1,3 and 1,6 betaglucans, 3% mineral matter and 35% dry matter.

The dilution began with Bio Imune, which was diluted in a ratio of 1:200 with water. Immediately after, a sample was collected from each of the 7 treatments for the initial bacterial count.

Next, six treatments received the different compositions of FOS and cell extract. An addition equivalent to 300 g of DM (Dry Matter) per 200 liters of water was calculated, i.e., 300 g DM of the composition per hectare.

The treatments were kept for 48 hours, under agitation on a shaker table at room temperature.

The bacterial counts were taken at the initial moment, immediately after dilution, after 24 hours and after 48 hours of agitation.

Table 4 below shows the bacterial counts of each of the treatments, measured in CFU/ml.

**TABLE 4**

| **Treatment** | **Composition (% DM)** | | ***Bacillus* population (CFU/ml)** | | |
|---|---|---|---|---|---|
| | **FOS** | **Cell extract** | **Initial** | **24 h** | **48 h** |
| T1 | 0% | 100% | 14,000,000 | 18,000,000 | 19,000,000 |
| T2 | 20% | 80% | 14,000,000 | 25,000,000 | 27,000,000 |
| T3 | 40% | 60% | 14,000,000 | 37,000,000 | 39,000,000 |
| T4 | 60% | 40% | 14,000,000 | 45,000,000 | 49,000,000 |
| T5 | 80% | 20% | 14,000,000 | 48,000,000 | 53,000,000 |
| T6 | 100% | 0% | 14,000,000 | 14,000,000 | 14,000,000 |
| Control | 0% | 0% | 14,000,000 | 13,000,000 | 12,000,000 |

The results in Table 4 show that treatments T4 and T5 were those that presented the best rate of multiplication of bacteria. Certainly because they have a better proportion between energy and proteins for multiplication of bacteria. In other words, the composition of this invention presents greater efficiency in terms of multiplication of *Bacillus* when the proportion of FOS is in the range of 40 and 80%, more specifically in the range between 60 and 85%.

The results in Table 4 also show that, between 24 and 48 hours of agitation, treatments T1 to T5 provided an extra growth of the *Bacillus* populations, which indicates that the composition also provides an increase in the longevity of the bacterial populations. This did not happen with the control treatment and T6, in which there was no source of protein for the bacteria to multiply.

Table 5 below shows the variation in the *Bacillus* populations of treatments T1 to T6 in relation to the control treatment. Mainly, treatments T3, T4 and T5 showed increases in bacterial populations of around 200% or more.

**TABLE 5**

| **Treatment** | **Variation of *Bacillus* Population** | | |
|---|---|---|---|
| | **24 h/initial** | **24 h/48 h** | **48 h/initial** |
| T1 | 29% | 6% | 36% |
| T2 | 79% | 8% | 93% |
| T3 | 164% | 5% | 179% |
| T4 | 221% | 9% | 250% |
| T5 | 243% | 10% | 279% |
| T6 | 0% | 0% | 0% |
| Control | -7% | -8% | -14% |

This is a strong indicator that the surface of plants sprayed with Bio Imune *Bacillus,* added to the composition of the present invention, will have much larger and longer-lasting populations of these good bacteria. Thus, they will have much more intense and prolonged protection against fungi and bacteria. This is an important economic advantage, since the composition of this invention is much cheaper than Bio Imune and other biodefensives containing *Bacillus.* Therefore, it allows them to be replaced, providing cost reduction.

Another consequence is the possibility of carrying out fewer sprayings during the crop cycle to obtain the same protection.

### EXAMPLE 2

The aim was to simulate the effects of partially replacing biodefensives containing bacteria of the *Bacillus* genus with the composition of this invention, in preventing the fungal disease known as Asian rust in soybean crops. This is considered the main threat to soybean crops in Brazil, which is the largest producer of this legume.

Since it is practically impossible to accurately measure the population of *Bacillus* bacteria on the leaves of plants, it was decided to dilute the Bio Imune product in the laboratory and culture the bacteria *in vitro,* comprising 10 treatments, in culture media containing 2 doses of the composition and 5 doses of the Bio Imune product, which is a biofungicide based on bacteria *Bacillus subtilis.*

The doses used in each treatment are shown in Table 6 below. The commercial formulation used was Bio Imune, manufactured by the company Vittia, composed of bacteria *Bacillus subtilis* BV02, recommended for combating the fungus *Phakospsora pachrhizi,* which causes Asian rust disease in soybean crops. The recommended use is 1.0 liter of product per ha/application, for a total of 4 applications per crop cycle. The dilution of the solution is 1:200, in order to make applications of 200 I/ha. The concentration of the product announced by the manufacturer was 3 × 10⁹.

The composition of this invention used in the experiment was composed of 70% FOS, produced by the company Yessinergy, having a concentration of 88%, 27% cell extract of *Saccharomyces cerevisae* yeast, containing 41% crude protein, 11% of 1,3 and 1,6 betaglucans, and the addition of 3% of a blend of chelated minerals. All percentages are on a dry matter basis. The final composition had 34% dry matter.

Initially, the different dosages of Bio Imune were diluted in a ratio of 1:200, in all 10 treatments, and packaged in bottles containing 400 ml of water each. The bottles were then shaken for 15 minutes and the first samples were collected to count the *Bacillus* bacteria in each treatment. The next step was to add the different dosages of the composition of this invention to the vials of each treatment, except for the control treatment, which consisted only of water and the Bio Imune product.

Subsequently, samples were collected from each treatment, after 24 hours and 48 hours of shaking, to count the *Bacillus* populations of each treatment.

The vials of all treatments remained on a shaking table (shaker) for 48 hours.

Table 6 below indicates that all treatments where the composition of this invention was used showed significantly larger *Bacillus* populations, regardless of the reduction in the dosage of Bio Imune that was tested. This indicates that the composition was highly efficient in multiplying and increasing the longevity of populations of these good bacteria.

**TABLE 6**

| ***Bacillus* Inoculum l/ha** | **Composition l/ha** | ***Bacillus* Population CFU/ml** | | | **Sprayings per soybean cycle** | **Costs (US$/ha)** | |
|---|---|---|---|---|---|---|---|
| | | **Initial** | **24 h** | **48 h** | | **One spraying** | **All sprayings** |
| 1.0 | 0.0 | 14,000,000 | 13,500,000 | 12,000,000 | 4 | 24 | 96 |
| 1.0 | 1.0 | 14,000,000 | 47,000,000 | 49,000,000 | 2 | 30 | 60 |
| 0.7 | 1.0 | 10,000,000 | 34,000,000 | 36,000,000 | 2 | 24 | 48 |
| 0.7 | 1.5 | 10,000,000 | 47,000,000 | 49,000,000 | 2 | 27 | 54 |
| 0.5 | 1.0 | 7,000,000 | 27,000,000 | 28,000,000 | 3 | 20 | 60 |
| 0.5 | 1.5 | 7,000,000 | 36,000,000 | 38,000,000 | 2 | 23 | 46 |
| 0.3 | 1.0 | 4,000,000 | 20,000,000 | 21,000,000 | 3 | 16 | 48 |
| 0.3 | 1.5 | 4,000,000 | 29,000,000 | 30,000,000 | 3 | 19 | 57 |
| 0.1 | 1.0 | 1,000,000 | 14,000,000 | 15,000,000 | 4 | 12 | 48 |
| 0.1 | 1.5 | 1,000,000 | 21,000,000 | 22,000,000 | 3 | 15 | 45 |

There was no concern about repeating treatments, since FOS, which is the source of food energy in the composition of the present invention, is a saccharide that can only be fermented by bacteria that produce the extracellular invertase enzyme, which is the case of *Bacillus.* In other words, there was practically no risk of infection of the treatments by other bacteria.

The results in Table 6 show that adding the composition of this invention to Bio Imune, in a 1:1 ratio, produced a 3-fold increase in the *Bacillus* population. The treatment that simulated the use of only 10% of the recommended dose of the Bio Imune product, added with the composition of this invention, in the proportion of 1:10, also showed a final population of *Bacillus* greater than in the control treatment.

Since the price of the composition of this invention should be around 30% of the biodefensives based on *Bacillus,* such as Bio Imune, there is an obvious advantage in partial replacement. Furthermore, due to the large populations of *Bacillus* shown in the treatments that received the composition, even after 48 hours, it is possible to conclude that fewer sprayings will be necessary during the crop cycle.

Table 6 shows that 4 treatments would allow a reduction from four to two applications per cycle, that is, the composition of this invention can benefit agricultural producers with up to 50% cost reduction in controlling Asian rust in soybean crops.

Despite the example above, the use of the composition is not restricted to soybean crops or Asian rust disease, but extends to all crops and diseases, fungal or bacterial, that can be controlled with the use of bacteria of the *Bacillus* genus.

In fact, empirical evidence indicates that the isolated spraying of the composition of the present invention would already be sufficient to control several diseases, only with the multiplication of populations of autochthonous bacteria of the *Bacillus* genus.

### EXAMPLE 3

In example 3, it was evaluated the potentialization of *Beauveria bassiana* through the invention, acting as a Biological Pest Control Agent in sugarcane cultivation. *Beauveria bassiana* is an entomopathogenic fungus known for its effectiveness in controlling agricultural pests. This study was conducted to evaluate its potential as a biological control agent for pests affecting sugarcane crops, in the presence and absence of the enhancer based on extract of *Saccharomyces cerevisiae* and FOS.

The experimental site was the experimental area of Fazenda Bandeirantes located in Salmourão, SP, Brazil, having a history of infestation by sugarcane borer. The soil was prepared according to typical agricultural practices for sugarcane cultivation in the region. The Control Group and Experimental Group were established, each having multiple replicated doses. The Control Group did not receive standard market application of *Beauveria bassiana,* while the Experimental Group was treated with the fungus added at 4 ml per liter of the solution having the microorganism.

The fungus was applied directly to the sugarcane plants and to the soil around the plants, following the dosage and application instructions. During the sugarcane growth period, regular observations of the plants and monitoring of target pests were carried out. Parameters such as pest incidence, plant health, plant height, tillering production, among others, were recorded.

The data collected were statistically analyzed to compare pest incidence and plant performance in the Control and Experimental groups.

A significant reduction in the incidence of the target pest was observed in the Experimental Group compared to the Control, which proves that the compound increased the population of the biological agent that acts as an agricultural defensive.

Plants treated with *Beauveria bassiana* showed better vigor, more uniform height and greater production of ears compared to untreated plants.

This study highlights the potential of the invention as a potentiator of the biological agent *Beauveria bassiana* as an effective tool in the integrated management of pests in sugarcane cultivation, offering a more sustainable and environmentally friendly approach for farmers.

## Claims

1. Composition to promote the multiplication and survival of microorganisms, **characterized in that** it comprises fructo-oligosaccharides (FOS) and cell extract of *Saccharomyces cerevisae,* and also comprises a mass concentration of 1.3 and 1.6 Betaglucans ranging from 1% to 15%, wherein such betaglucans come from the cell extract, or have already been added in the form of cell wall.

2. Composition to promote the multiplication and survival of microorganisms, according to claim 1, **characterized in that** said microorganisms comprise fungi or bacteria such as the genera *Bacillus spp., Rhizobium spp., Azospirillum spp., Azotobacter, Pseudomonas spp., Allorhizobium spp., Azorhizobium, Bradyrhizobium* and *Streptomyces spp.,* the fungi *Metarhizium spp., Beauveria spp. and Trichoderma spp.,* in addition to the mycorrhizal fungi *Glomus spp., Rhizophagus spp., Funneliformis spp., Amanita spp., Boletus spp.* and *Laccaria spp..*

3. Composition to promote the multiplication and survival of microorganisms, according to claim 1, **characterized in that** said composition can be applied to the aerial and underground epithelium of cultivated plants.

4. Composition to promote the multiplication and survival of microorganisms, according to claim 1, **characterized in that** said microorganisms may be autochthonous or have been sprayed on the plant.

5. Composition to promote the multiplication and survival of microorganisms, according to claim 1, **characterized in that** said composition comprises, by dry matter mass, 10% to 90% fructo-oligosaccharides (FOS) and 10% to 90% cell extract of *Saccharomyces cerevisae,* with the concentration of 1.3 and 1.6 Betaglucans being between 1% and 15%, said betaglucans coming from the cell extract or already added in the form of cell wall.

6. Composition to promote the multiplication and survival of microorganisms, according to claim 5, **characterized in that** said composition comprises, in dry matter, 15% to 90% fructo-oligosaccharides (FOS) and 15% to 90% cell extract of *Saccharomyces cerevisae,* with the concentration of 1.3 and 1.6 Betaglucans being between 2% and 15%, said betaglucans coming from the cell extract or already added in the form of cell wall.

7. Composition to promote the multiplication and survival of microorganisms, according to claim 6, **characterized in that** said composition comprises, in dry matter, 20% to 90% fructo-oligosaccharides (FOS) and 20% to 90% cell extract of *Saccharomyces cerevisae,* with a concentration of 1.3 and 1.6 Betaglucans between 2% and 15%, said betaglucans coming from the cell extract or already added in the form of cell wall.

8. Composition to promote the multiplication and survival of microorganisms, according to claim 1, **characterized in that** said composition can be presented in the form of powder or liquid.

9. Composition to promote the multiplication and survival of microorganisms, according to claim 8, **characterized in that** in powder form it has a moisture content of less than 5% and can be diluted in water for spraying on plants.

10. Composition to promote the multiplication and survival of microorganisms, according to claim 8, **characterized in that** said composition can be presented in liquid form, comprising by mass from 20% to 50% dry matter.

11. Composition to promote the multiplication and survival of microorganisms, according to claim 1, **characterized in that** optionally said composition may be enriched with betaglucans and minerals.

12. Composition to promote the multiplication and survival of microorganisms, according to claim 1, **characterized in that** said fructo-oligosaccharide (FOS) comprises any proportion between its oligomers GF2 (1 Glucose + 2 Fructoses), GF3, GF4, GF5 and GF6.

13. Composition to promote the multiplication and survival of microorganisms, according to claim 12, **characterized in that** the fructooligosaccharide (FOS) has a greater proportion of longer chain oligomers, GF4, GF5 and GF6.

14. Composition to promote the multiplication and survival of microorganisms, according to claim 1, **characterized in that** the cell extract of Saccharomyces cerevisae is a residue or waste from ethanol production.

15. Method for treating plants, according to the composition described in any of the preceding claims, **characterized in that** the dosage of the composition of dry matter mass, recommended for the application by spraying it on plants or soil, can vary between 0.20 kg/ha and 1.00 kg/ha.

16. Method for treating plants, using the composition, according to claim 15, **characterized in that** the composition can be used mixed with the biodefensive containing microorganisms, fungi, or bacteria chosen from the group comprising: genera Bacillus spp, Rhizobium spp, Azospirillum spp, Azotobacter, Pseudomonas spp, Allorhizobium spp, Azorhizobium, Bradyrhizobium and Streptomyces spp, the fungi Metarhizium spp and Beauveria spp and Trichoderma spp, in addition to the mycorrhizal fungi Glomus spp, Rhizophagus spp, Funneliformis spp, Amanita spp, Boletus spp and Laccaria spp.

17. Method for treating plants with the composition according to claims 15 or 16, **characterized in that** said composition is sprayed alone on the plants, without being mixed with biodefensive products containing microorganisms, fungi, or bacteria chosen from the group comprising: genera Bacillus spp, Rhizobium spp, Azospirillum spp, Azotobacter, Pseudomonas spp, Allorhizobium spp, Azorhizobium, Bradyrhizobium and Streptomyces spp, the fungi Metarhizium spp and Beauveria spp and Trichoderma spp, in addition to the mycorrhizal fungi Glomus spp, Rhizophagus spp, Funneliformis spp, Amanita spp, Boletus spp and Laccaria spp.

18. Use of the composition to promote the multiplication and survival of microorganisms, according to any of the previous claims, **characterized in that** said use occurs in agriculture or agroindustry.

19. Use of the composition to promote the multiplication and survival of microorganisms, according to any of the previous claims, **characterized by** stimulating the growth and viability of microbial strains of bacteria involving the genera Bacillus spp, Rhizobium spp, Azospirillum spp, Azotobacter, Pseudomonas spp, Allorhizobium spp, Azorhizobium, Bradyrhizobium and Streptomyces spp, the fungi Metarhizium spp and Beauveria spp and Trichoderma spp, in addition to the mycorrhizal fungi Glomus spp, Rhizophagus spp, Funneliformis spp, Amanita spp, Boletus spp and Laccaria spp.
